# EUROPEAN PATENT APPLICATION

(11) **EP 3 165 098 A1**
(43) Date of publication of application: **10.05.2017**
(21) Application number: 14896986.8
(22) Date of filing: 19.08.2014
(51) Int. Cl.: A23L 3/015, A61L 2/02, A61L 2/04

(54) **METHOD OF STERILIZING OR INACTIVATING HEAT-RESISTANT SPORE-FORMING BACTERIA**

(30) Priority: 05.07.2014 JP 2014139222
(71) Applicant: Echigoseika Co. Ltd., Nagaoka-shi, Niigata 940-8622 (JP)
(72) Inventor: OGINO Miyuki, Niigata-shi Niigata 950-2181 (JP); NISHIUMI Tadayuki, Niigata-shi Niigata 950-2181 (JP); KOBAYASHI Atsushi, Ojiya-shi Niigata 947-0193 (JP); YAMAZAKI Akira, Ojiya-shi Niigata 947-0193 (JP); OHARA Eri, Ojiya-shi Niigata 947-0193 (JP); KAWAMURA Mariko, Ojiya-shi Niigata 947-0193 (JP); HOSHINO Jun, Ojiya-shi Niigata 947-0193 (JP)
(74) Representative: Hoffmann Eitle
(86) International application number: PCT/JP2014/071620
(87) International publication number: WO 2016/006121

(57) **Abstract**

Provided is an innovative method for sterilization or inactivation treatment of heat-resistant spore-forming bacteria that demonstrates a sufficient sterilization effect in a reliable manner and that can be implemented using simple equipment by reducing the heat resistance of heat-resistant spore-forming bacteria by the effect of decompression treatment in a high-pressure process, and by carrying out a heat sterilization treatment after a predetermined resting time has elapsed after the decompression to thereby sterilize and inactivate heat-resistant bacteria that have reduced heat resistance. A method for sterilizing or inactivating heat-resistant spore-forming bacteria by performing, as a pretreatment of a heat sterilization treatment, a high-pressure treatment in which a hydrostatic pressure of 50 MPa or more and 200 MPa or less is applied to an object under a temperature of 50°C or less, and a decompression treatment for decompression is carried out, after which the object having undergone the decompression treatment is subjected to the heat sterilization treatment after a predetermined resting time has elapsed after a variation in hydrostatic pressure by the decompression has occurred.

## Description

### TECHNICAL FIELD

The present invention relates to a method of sterilizing or inactivating heat-resistant spore-forming bacteria using elapsed time after decompression treatment (hydrostatic pressure variation).

### BACKGROUND ART

Heat-resistant microorganisms that inhabit the natural world live in harvested agricultural and marine products or are added to and live in processed foods obtained using these agricultural and marine products as raw materials, and have a negative effect on long term storage.

Many of these heat-resistant microorganisms are difficult to sterilize using reagents, electron beam, radiation, pressure sterilization, heat sterilization, and the like, and, at present, sterilization is carried out by pressure and heat sterilization (hereinafter referred to as retort sterilization) at 121°C or more.

This retort sterilization is carried out for canned and pouched food products, but the original taste, aroma, and nutrients of the food product are destroyed or otherwise lost, and the food texture that existed prior to sterilization is often lost.

Moreover, sterilization of heat-resistant bacteria using pressure has been proposed using methods such as:
(1) applying a high pressure of 400 MPa or higher;
(2) applying pressure using the effects of heat processing (methods that simultaneously apply heat and pressure);
(3) applying heat immediately after a heating process; and
(4) applying an additive to adjust the pH (hydrogen ion concentration), and applying pressure to enhance sterilization effect.

However, method (1) is incapable of sterilizing heat-resistant bacteria and long term storage is therefore difficult, methods (2) and (3) perform sterilization using heat effects rather than pressure effects and ultimately food product quality is sacrificed in similar fashion to retort sterilization (pressure and heat sterilization), and in method (4), taste is sacrificed by an additive and the originally desired taste of a food product cannot be obtained by the sterilization process. These and other problems, as a result, make it impossible to sterilize heat-resistant bacteria using pressure and to manufacture a sterile food product that for which long term storage is possible.

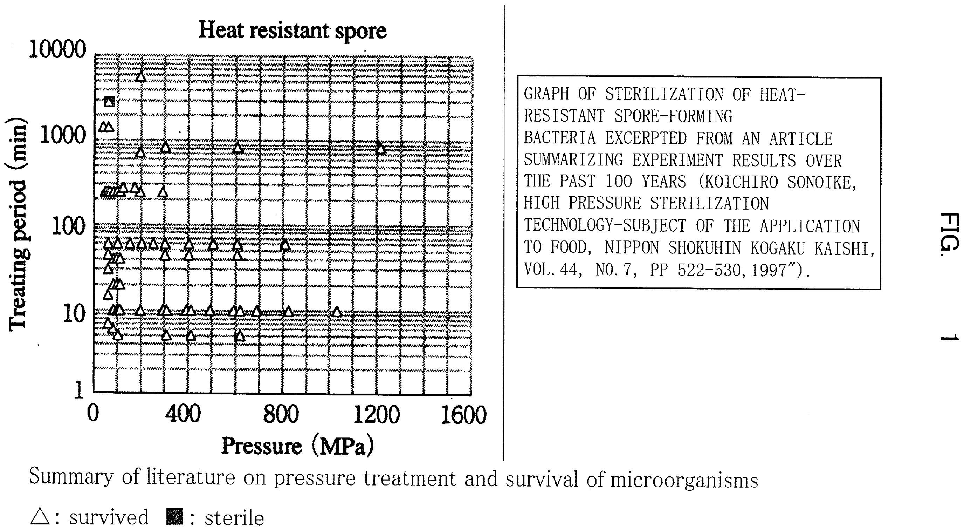

Koichiro Sonoike published a summary of literature on pressure treatment and survival of microorganism over the past 100 years (Koichiro Sonoike, "Seibutsu-kogaku Vol. 91, No. 2, pp 50-72, 2013"), and FIG. 1 shows results that indicate that conventionally assumed sterilization effect of pressure treatment alone is poor, and sterilization by a high pressure of 1200 MPa is not possible for heat-resistant spore-forming bacteria in particular.

In other words, as described above, sterilization of heat-resistant spores is difficult with high-pressure treatment alone, and even with a combined effect of heat treatment and pressure treatment (an effective combination for sterilization), an effective temperature is required for heat treatment alone (a temperature capable of sterilization without depending on the effect of pressure), and when consideration is given to the cost of pressure device, sterilization conditions at 121°C or less and 200 MPa or less in which production facilities are relatively low cost have not be found.

For example, various patent applications have been made for techniques or other methods for sterilization by heat treatment immediately after high-pressure treatment, but all require high-cost equipment. In other words, a sufficient sterilizing effect at 200 MPa or less has not been achieved with an inexpensive device and low operating costs, implementation is still viewed as being difficult, and there is a strong need for further research and results (Japanese Laid-open Patent Application No. 2004-81036).

### [Prior Art Documents]

### [Patent Documents]

[Patent Document 1] Japanese Laid-open Patent Application No. 2004-81036
[Non-patent Document 1] SONOIKE, Koichiro, "Seibutsu-kogaku, Vol. 91, No. 2, pp 50-72, 2013"

### DISCLOSURE OF THE INVENTION

### [Problem the Invention is to Solve]

The applicant carried out further research under the current state of the art, perfected the present invention having learned through research and consideration of the mechanisms of heat resistance of heat-resistant spore-forming bacteria and through measurement thereof from the correlation with the turbidity of a spore suspension and the relationship between heat resistance and the passage of time after decompression treatment, and found conditions in which heat-resistant spore-forming bacteria can be efficiently sterilized or inactivated using a simple technique with the independent effects of a decompression treatment at a relatively low pressure 200 MPa or less and a heat sterilization treatment of 121°C or less.

In other words, an object of the present invention is to provide a method for imparting a hydrostatic pressure variation to an object to reduce the heat resistance of heat-resistant spore-forming bacteria by the variation, specifically, the effect of decompression treatment in high-pressure treatment, and thereafter performing a heat sterilization treatment after a predetermined resting time has elapsed after the decompression (after 10 minutes or more have elapsed and before 18 hours or more have elapsed, e.g., after elapse of time required for the turbidity of a spore suspension, which is an index of heat resistance after the decompression treatment, to be 60% or less of the value immediately after the decompression) to thereby sterilize and inactivate heat-resistant bacteria that have reduced heat resistance.

In other words, in view of the effect of decompression treatment after high-pressure treatment in order to enhance the sterilization effect in a range of simple use of pressure and temperature without dependence on high pressure and high temperature, which have been conventionally held to allow sterilization by the independent effects of decompression treatment and heat sterilization treatment, the inventor found that, *inter alia*, the above-stated problem is solved by placing emphasis on hydrostatic pressure variation by decompression treatment, that heat resistance of heat-resistant spore-forming bacteria is sufficiently reduced together with time even with the above-stated pressure level, and that sufficient effect can be obtained with heat sterilization at a temperature level (level of temperature increase) such as described above while heat resistance is low for a predetermined length of time, and an object of the present invention is to provide an innovative method for sterilization or inactivation treatment of heat-resistant spore-forming bacteria that demonstrates a sufficient sterilization effect in a reliable manner and that can be implemented using simple equipment.

### [Means for Solving the Problem]

The main points of the present invention are described below with reference to the attached drawings.

A first aspect of the present invention relates to a method for sterilizing or inactivating heat-resistant spore-forming bacteria, characterized in that, as a pretreatment of a heat sterilization treatment, a high-pressure treatment is carried out in which a hydrostatic pressure of 50 MPa or more and 200 MPa or less is applied to an object under a temperature of 50°C or less, a decompression treatment for decompression is carried out, after which the object having undergone the decompression treatment is subjected to the heat sterilization treatment, in which a temperature of at least 121°C or less, which is a temperature of 50°C to 80°C above the initial temperature, is maintained for 10 minutes or longer, after 10 minutes or more have elapsed and before 18 hours or more have elapsed after a variation in hydrostatic pressure has occurred by the decompression.

A second aspect of the present invention also relates to the method for sterilizing or inactivating heat-resistant spore-forming bacteria according to the first aspect, characterized in that the time at which the turbidity of a spore suspension, which is an index of heat resistance after the decompression treatment, is 60% or less of the value immediately after the decompression is ascertained as a resting time by measurement in advance, a pause is made until the resting time has elapsed immediately after the decompression, and the heat sterilization treatment is carried out.

A third aspect of the present invention also relates to the method for sterilizing or inactivating heat-resistant spore-forming bacteria according to the first or second aspect, characterized in that the object is harvested agricultural and marine products, processed foods processed using these products, or animal organs, blood, animal cell tissue, plant cell tissue, or seeds.

### [Effects of the Invention]

The present invention, being configured as described above, is a method for sterilizing or inactivating heat-resistant spore-forming bacteria capable of efficiently sterilizing or inactivating heat-resistant spore-forming bacteria using a simple technique by the independent effects of a decompression treatment at a relatively low pressure of 200 MPa or less and heat sterilization treatment at 121°C or less.

In other words, the present invention provides an innovative method for sterilization or inactivation treatment of heat-resistant spore-forming bacteria that demonstrates a sufficient sterilization effect in a reliable manner and that can be implemented using simple equipment by imparting a hydrostatic pressure variation to an object to reduce the heat resistance of heat-resistant spore-forming bacteria by the variation, specifically, the effect of decompression treatment in high-pressure treatment, and thereafter performing a heat sterilization treatment after a predetermined resting time has elapsed after the decompression (after 10 minutes or more have elapsed and before 18 hours or more have elapsed, e.g., after elapse of time required for the turbidity of a spore suspension, which is an index of heat resistance after the decompression treatment, to be 60% or less of the value immediately after the decompression) to thereby allow heat-resistance bacteria having reduced heat resistance to be sterilized and inactivated.

Therefore, the following effects are obtained in accordance with the present invention.
(1) The required decompression pressure is low; i.e., 50 MPa or more and 200 MPa or less, so the pressure device is inexpensive, equipment costs are low, and application can be made to processing of foods that have low profit margins.
(2) The time required until heat sterilization treatment is carried out after decompression treatment is about 10 minutes or more and 18 hours or less, and the method is therefore advantageous in that there is leeway in the time until sterilization is carried out, and there are few short-time restrictions leading to the next step.
(3) Reduced heat resistance of heat-resistant spore-forming bacteria is induced by decompression, which is a physical factor, and the elapse of time thereafter, biological germination is therefore unlikely to proceed even if the object is left standing, and microorganism-related contamination of the object does not occur.
(4) The pressure of decompression treatment is 50 MPa or greater by hydrostatic pressure, a relatively small variation in pressure can be used in the step rather than a pressurization reference (rather than maximum pressurization), and the process can proceed to a subsequent processing step with residual pressure.
(5) The temperature required for heat treatment is lower than the temperature of conventional retort sterilization (pressurized heat sterilization), so implementation is simple. Since the low temperature and low pressure allow a common commercially available high-temperature pipe to be used, the various treatments of the present invention can be continuously carried out in a pipeline for food products or the like that are handled as a fluid, and the manufacturing equipment and running costs are low.
(6) Enzymes contained in a food product often cause changes in the food product in long-term storage, which creates the need for frozen preservation even when microorganisms are not present, and enzyme deactivation becomes a problem. Since the method of the present invention allows for a concurrent enzyme "deactivation step," equipment for preservation in refrigeration is thereafter not required, making it possible to manufacture an easy-to-handle product.
(7) Pressure devices can be operated in alternating fashion as two lines when batch-type pressure treatment is to be carried out, pressure can be recovered while continuous processing is maintained, and about half the pressure energy can be recovered, thereby achieving energy savings. Furthermore, using the time of decompression as a reference facilitates ascertainment of the moment of decompression, repeated cycle time is easy to determine, and managing the frequency of work steps becomes advantageous in terms of time.

### BRIEF DESCRIPTION OF THE DRAWINGS

FIG. 1 is a graph summarizing an article reporting the survival numbers of bacteria when heat-resistant spore-forming bacteria are subjected to a high-pressure treatment in "High Pressure Sterilization Technology - Subject for the Application to Food;"
FIG. 2 is a view illustrating the structure of spore;
FIG. 3 is a graph showing the relationship between germination and change in turbidity as published in Seibutsu-kogaku Vol. 91, No. 2 (50-72, 2013), and shows the relationship between photomicrographs of spores and change in turbidity;
FIG. 4 is a graph showing change in turbidity after decompression treatment of *B. cereus* spores of the present example;
FIG. 5 is a graph showing the recovery time of the heat resistance of *B. cereus* spores reduced by the decompression treatment of the present example;
FIG. 6 is a graph showing change in turbidity of the present example after decompression treatment (200 MPa, 25°C, 10 minutes) of *B. cereus* suspended in a 0.067-M phosphate buffer solution;
FIG. 7 is a graph showing change in bacteria survival numbers of the present example in decompression treatment of *Bacillus subtilis* (NBRC 3134) spores suspended in a 0.067-M phosphate buffer solution, and in a subsequent heat treatment (90°C);
FIG. 8 is a graph showing change in bacteria survival numbers of the present example in decompression treatment of *Bacillus subtilis* (NBRC 3134) spores suspended in a 0.067-M phosphate buffer solution, and in a subsequent heat treatment (100°C);
FIG. 9 is a graph showing change in bacteria survival numbers of the present example in decompression treatment of *Bacillus subtilis* (NBRC 3134) spores suspended in a 0.067-M phosphate buffer solution, and in a subsequent heat treatment (110°C);
FIG. 10 is a graph showing change in bacteria survival numbers of the present example in decompression treatment of *Bacillus cereus* (NBRC 13494) spores suspended in a 0.067-M phosphate buffer solution, and in a subsequent heat treatment (90°C);
FIG. 11 is a graph showing change in bacteria survival numbers of the present example in decompression treatment of *Bacillus cereus* (NBRC 13494) spores suspended in a 0.067-M phosphate buffer solution, and in a subsequent heat treatment (100°C);
FIG. 12 is a graph showing change in bacteria survival numbers of the present example in decompression treatment of *Bacillus cereus* (NBRC 13494) spores suspended in a 0.067-M phosphate buffer solution, and in a subsequent heat treatment (110°C);
FIG. 13 is a table showing turbidity after decompression treatment (200 MPa, 25°C, 1 minute) of *B. cereus* spores, and the survival rate in heat sterilization (100°C, 30 minutes) after decompression treatment of the present example;
FIG. 14 is a graph showing the relationship between turbidity after decompression treatment (200 MPa, 25°C, 1 minute) of *B. cereus* spores, and the survival rate in heat sterilization (100°C, 30 minutes) after decompression treatment of the present example;
FIG. 15 is a graph showing the change in turbidity of the present example after decompression treatment of *Bacillus cereus* (NBRC 13494) spore-forming bacteria liquid;
FIG. 16 is a graph showing change in bacteria survival numbers of the present example after a Kagoshima Prefecture sweet potato (beni-azuma, unpeeled, round slices) had been subjected to decompression treatment (200 MPa, 50°C, 5 minutes) followed 10 minutes later by heat sterilization (100°C);
FIG. 17 is a graph showing change in bacteria survival numbers of the present example after a Kumamoto Prefecture sweet potato (beni-haruka, round slices) had been subjected to decompression treatment (200 MPa, 50°C, 5 minutes) followed 10 minutes later by heat sterilization (100°C, 30 minutes);
FIG. 18 is a graph showing change in bacteria survival numbers of the present example after a Kagoshima Prefecture potato (round slices) had been subjected to decompression treatment (200 MPa, 25°C, 2 minutes) followed 10 minutes later by heat sterilization (100°C, 15 minutes; 105°C, 15 minutes);
FIG. 19 is a graph showing change in bacteria survival numbers of the present example in a frozen takoyaki (octopus dumpling) which was subjected to decompression treatment (200 MPa, 25°C, 2 minutes) followed 10 minutes later by heat sterilization (105°C, 30 minutes), and then refrigeration;
FIG. 20 is a graph showing change in bacteria survival numbers of the present example in a hamburger steak which was subjected to decompression treatment (200 MPa, 25°C, 2 minutes) followed 10 minutes later by heat sterilization (100°C, 15 minutes; 105°C, 15 minutes), and then refrigeration;
FIG. 21 is a graph showing change in bacteria survival numbers of the present example in shumai (shao-mai) which was subjected to decompression treatment (200 MPa, 25°C, 2 minutes) followed 10 minutes later by heat sterilization (100°C, 15 minutes; 105°C, 15 minutes), and then refrigeration; and
FIG. 22 is a graph showing change in bacteria survival numbers of the present example in pineapple which was subjected to decompression treatment (200 MPa, 25°C, 2 minutes) followed 10 minutes later by heat sterilization (100°C, 15 minutes; 105°C, 15 minutes), and then refrigeration.

### BEST MODE FOR CARRYING OUT THE INVENTION

Preferred embodiments of the present invention are briefly described below with reference to the diagrams while indicating the effects of the present invention.

The mechanisms by which spores maintain heat resistance were unknown until recently, and it has been found that heat resistance is maintained roughly by two mechanisms. A first mechanism is that the spore coat, which is the surface of a spore cell, retains hydrophobicity, and therefore the contact angle with water is low, and the surface contact area is low. Consequently, it is difficult for heat to penetrate the interior by heat transfer, and the transfer of heat through the cortex to the core is blocked.

A second mechanism is that penetration of water from the exterior is blocked because the spore coat keeps its hydrophobicity, and penetration of water to the core and penetration of heat in accompaniment therewith are blocked. Therefore, the concentration of enzymes, protein, metal ions, and the like contained in the cortex and required to maintain life is kept within a tolerance range by osmotic pressure.

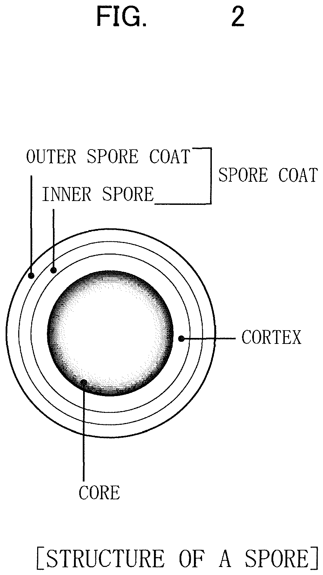

The present invention addresses the shared portion of the two mechanisms, i.e., that "the spore coat retains hydrophobicity," and is a method for implementing sterilization and inactivation by imparting hydrostatic pressure variation to reduce the hydrophobicity of the spore coat boundary, increasing water permeability, and facilitating penetration of water and heat to the interior of the spore. FIG. 2 shows the structure of a spore.

In other words, the invention provides a sterilization method in which sufficient sterilization effect is reliably demonstrated by using the phenomenon of reducing the heat resistance of microorganisms by the effect of decompression treatment instead of the effect of pressure treatment alone, reducing the heat resistance of heat-resistant spore-forming bacteria, and thereafter performing sterilization at a low temperature in a range that does not compromise food product quality.

In other words, the inventor addresses the shared portion of two defense mechanisms possessed by spores of heat-resistant bacteria, i.e., that "the spore coat retains hydrophobicity," and perfected the present invention having found that an important point is to implement sterilization and inactivation by imparting hydrostatic pressure variation to reduce the hydrophobicity of the spore coat boundary, increase water permeability, and facilitate penetration of water and heat to the interior of the spore. Having carried out research, experimentation, and measurements, the inventor found the conditions for achieving this method.

Another point perfecting the present invention is that change in heat resistance is ascertained by measuring the turbidity of a spore suspension (OD). In other words, an innovative method of sterilizing or inactivating heat-resistant spore-forming bacteria that is simple to implement and that demonstrates a sufficient sterilization effect was made having recognized the importance of resting time after decompression treatment in a highly practical heat sterilization treatment carried out at a relatively low high-pressure treatment and a relatively low temperature, and having specifically found the time required therefor.

More specifically, it is known that a reduction in turbidity of the spore solution (spore suspension) is an index of reduced heat resistance and is also a reference for determining the germination step. In other words, reduced turbidity is one important observation method that makes it possible externally ascertain the state in which sterilization can be carried out by high-pressure and decompression treatments, even when germination has not occurred. Determining the heat resistance of spores in this manner is important because cultivation work, which requires time and labor, can be omitted in order to ascertain whether microorganisms have been reduced in content after a heat sterilization treatment. In the present invention, a range was found in which sterilization effect can be sufficiently demonstrated in a reliable manner with the lowest possible temperature and even lower pressure by reducing the turbidity of a spore solution by decompression treatment resulting from a high-pressure treatment, and using this as an index of heat resistance.

Even more specifically, as described above, the portion shared by the two defensive mechanisms of spores of heat resistance bacteria, i.e., that "the spore coat retains hydrophobicity" is used to achieve sterilization and inactivation of spores by imparting hydrostatic pressure variation by decompression to thereby reduce the hydrophobicity of the spore coat boundary, increase water permeability, and facilitate penetration of water and heat to the interior of the spore.

Currently, there have been no cases reporting change in turbidity by the passage of time following high-pressure treatment, and the present invention, having solved the problem of ascertaining the conditions in which heat resistance changes together with the passage of time after decompression treatment resulting from high-pressure treatment, confirms a phenomenon in which turbidity is reduced in corresponding fashion to the passage of time, by detailed observation of spore solution changes generated inside a pressure container following decompression treatment in a high-pressure treatment.

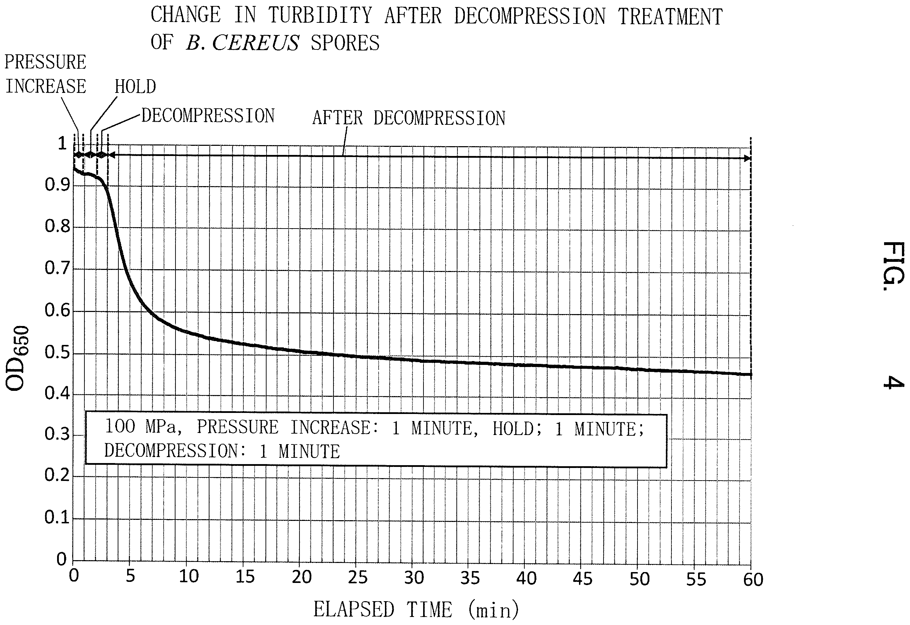

As shown in FIG. 4 to be later described, turbidity is rapidly reduced after decompression. The present invention for sufficiently sterilizing or inactivating heat-resistant spore-forming bacteria was made by sufficiently reducing the heat resistance of heat-resistant spore-forming bacteria in a fixed period of time (resting time) immediately following decompression treatment, and thereafter performing heat sterilization.

In other words, in view of the effect of decompression treatment after high-pressure treatment in order to enhance sterilization effect in a range of simple use of pressure and temperature without dependence on high pressure and high temperature, which have been conventionally held to allow sterilization by the independent effects of decompression treatment and heat sterilization treatment, the inventor found that, *inter alia*, previous problems are solved by placing emphasis on hydrostatic pressure variation resulting from decompression treatment, that heat resistance of heat-resistant spore-forming bacteria is sufficiently reduced together with time even with the above-stated pressure level, and that sufficient effect can be obtained with heat sterilization at a temperature level (level of temperature increase) such as described above while heat resistance is low for a predetermined length of time, and the present invention provides an innovative method for sterilization or inactivation treatment of heat-resistant spore-forming bacteria that demonstrates a sufficient sterilization effect in a reliable manner and that can be implemented using simple equipment.

### [Examples]

Specific examples of the present invention are described below with reference to the diagrams.

FIG. 1 shows a portion of an article published by Koichiro SONOIKE in relation to research over the past 100 years concerning sterilization effects when heat-resistant spore-forming bacteria are subjected to high-pressure treatment, and shows results that indicate that heat-resistant spore-forming bacteria cannot be sterilized by a high pressure of 1200 MPa.

Sterilization of microorganisms by high-pressure treatment greatly differs in effect between heat-resistant bacteria and non-heat-resistant bacteria.

In other words, it is known that the effect of high-pressure treatment on non-heat-resistant bacteria is dramatic, and that heating during pressurization is even more effective.

Therefore, there are a great many strains that die, including, e.g., most nutritive cells, Gram-negative bacteria, mold, and yeast.

However, as described above, it has been shown that sterilization of heat-resistant spore-forming bacteria is difficult even under a pressure of 400 MPa or more, and it has been found in follow-up research that sufficient sterilization effect cannot be demonstrated with a high-pressure treatment alone, or even with heating simultaneously or immediately after treatment unless the heating temperature is high.

In view of the above, the applicant carried out further research and consideration of the mechanisms of heat resistance of heat-resistant spore-forming bacteria, measured the turbidity of a spore suspension and the passage of time after decompression treatment in relation to heat resistance, and ascertained, in advance, the effects of heat sterilization treatment on sterilization. The present invention was perfected in finding the conditions in which heat-resistant spore-forming bacteria can be efficiently sterilized or inactivated using a simple technique with the independent effects of a decompression treatment at a relatively low pressure 200 MPa or less and a heat sterilization treatment of 121°C or less.

In other words, as a pretreatment of a heat sterilization treatment, a decompression treatment in which a hydrostatic pressure of 50 MPa or more and 200 MPa or less is applied to an object under a temperature of 50°C or less to perform high-pressure treatment and then decompression, after which, the object, having undergone decompression treatment, is subjected to the heat sterilization treatment in which a temperature of 50°C to 80°C greater (a temperature of at least 121°C or less) than the starting temperature is maintained for 10 minutes or longer, after 10 minutes or more have elapsed and before 18 hours or more have elapsed, in other words, immediately after decompression, i.e., immediately after a variation in hydrostatic pressure has occurred by the decompression.

In other words, as a pretreatment of a heat sterilization treatment, a decompression treatment in which a hydrostatic pressure of 50 MPa or more and 200 MPa or less is applied to an object under a temperature of 50°C or less to perform high-pressure treatment and then decompression, after which, the object, having undergone decompression treatment, is subjected to the heat sterilization treatment after a predetermined resting time has elapsed after a change in hydrostatic pressure has occurred due to the decompression. The predetermined resting time determined by pre-measurement is the time at which the turbidity of a spore suspension, which is an index of heat resistance after the decompression treatment, is 60% or less of the value immediately after the decompression. The determined time is used as the predetermined resting time, and the process waits for the resting time (about 10 minutes or more) to elapse starting immediately after the decompression to carry out the heat sterilization treatment.

Therefore, in view of the effect of decompression treatment after high-pressure treatment in order to enhance sterilization effect in a range of simple use of pressure and temperature without dependence on high pressure and high temperature, which have been conventionally held to allow sterilization by the independent effects of decompression treatment and heat sterilization treatment, the inventor found that, *inter alia*, previous problems are solved by placing emphasis on hydrostatic pressure variation resulting from decompression treatment, that heat resistance of heat-resistant spore-forming bacteria is sufficiently reduced together with time even with the above-stated pressure level, and that sufficient effect can be obtained with heat sterilization at a temperature level such as described above while heat resistance is low for a predetermined length of time, and the present invention provides an innovative method for sterilization or inactivation treatment of heat-resistant spore-forming bacteria that demonstrates a sufficient sterilization effect in a reliable manner and that can be implemented using simple equipment.

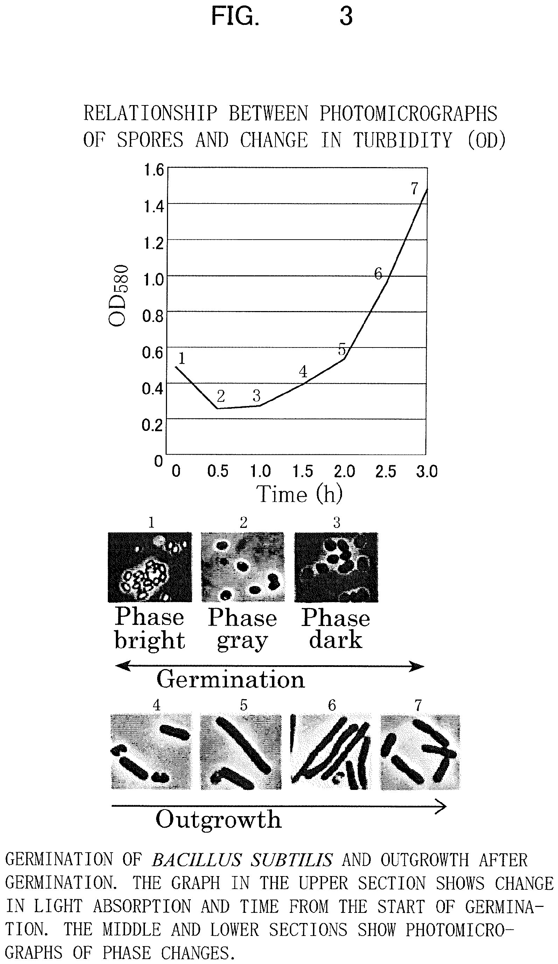

FIG. 3 is a graph showing the relationship between germination and change in turbidity as published in Seibutsu-kogaku Vol. 91, No. 2 (50-72, 2013), and shows the relationship between photomicrographs of spores and change in turbidity (OD).

FIG. 4 shows the results of an experiment involving change in turbidity after decompression treatment of *B. cereus* spores.

In other words, FIG. 4 shows the steps carried out in the high-pressure treatment step, wherein pressure is increased to 100 MPa (pressure increase time: 1 minute), held for one minute, and the decompressed to 0.1 MPa (decompression time: 1 minute), i.e., the change in turbidity (OD: optical density) after high-pressure treatment (pressure increase, pressure hold), decompression treatment (decompression), and decompression.

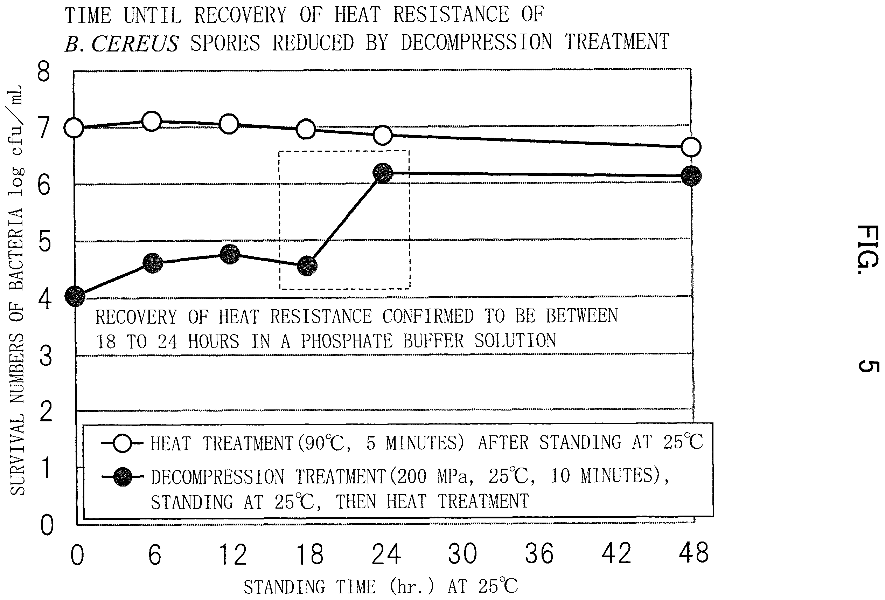

First, it is apparent from the change in turbidity of *Bacillus subtilis* spores as seen in FIG. 3 that turbidity is about 0.3 for spores of photographs 2 and 3 darkening prior to germination, and these spores do not have heat resistance. This generally shows that turbidity has decreased prior to germination and that heat resistance is decreased. However, it has been found that turbidity rapidly decreases, as shown in FIG. 5, after decompression obtained by a high-pressure treatment, and that heat resistance is reduced.

As discussed above, it is critical to measure OD as an index of heat resistance, and as of this writing, changes in OD over actual time have not been measured from the start of decompression or under high pressure. In the present invention, change in OD after decompression treatment that follows high-pressure treatment is measured together with the passage of time as means for confirming reduced heat resistance, and a decrease in heat resistance was confirmed thereby.

When the graph of FIG. 4 is observed by step, the change in OD can be confirmed to rapidly decrease from decompression in comparison with one minute of pressure increase at 100 MPa, and holding. In other words, it was found that a reduction in heat resistance of the spores is very greatly reduced by the passage of time starting from immediately after decompression in comparison with at the start of pressurization.

The graph of OD shown in FIG. 4 shows on the vertical axis the measurement value of OD₆₅₀ (optical density at a wavelength of 650 nanometers) rather than the amount of change in OD.

The initial bacteria count was adjusted to about 10⁸, and the experiment was carried out at 0.9 to 1.0 at this point.

Turbidity is reduced if the concentration of spore liquid is reduced, and the level is ordinarily adjusted to about 0.6, but the experiment was carried out with the level intentionally slightly high with the idea of measuring the degree of reduction in the steps of pressure increase, hold, decompression, and after decompression.

In the materials indicated in FIG. 3 (Seibutsu-kogaku Vol. 91, No. 2, pp 50-72, 2013), 0.6 is Phase-gray, and in Phase-dark the level was reduced by about half to 0.3.

When the initial value is set to 0.9 even with data obtained by measuring change in turbidity after decompression as shown in FIG. 4, the value was half at about 0.4 at the end point after decompression.

In the present embodiment, 10 minutes, which is when the turbidity value after decompression treatment is about 60%, was set as the required resting time, and it was confirmed that sufficient sterilization effect was demonstrated even with low-temperature sterilization by performing heat sterilization after 10 minutes or more had elapsed.

More specifically, it is known that a reduction in turbidity of the spore solution (spore suspension) is an index of reduced heat resistance as described above, and is also a reference for determining the germination step. In other words, reduced turbidity is one important observation method that makes it possible externally ascertain the state in which sterilization can be carried out by high-pressure and decompression treatments, even when germination has not occurred. Determining the heat resistance of spores in this manner is important because cultivation work, which requires time and labor, can be omitted in order to ascertain in advance whether microorganisms have been reduced in content after a heat sterilization treatment. In the present invention, a range was found in which sterilization effect can be sufficiently demonstrated in a reliable manner with the lowest possible temperature and even lower pressure by reducing the turbidity of a spore solution by decompression treatment resulting from a high-pressure treatment, and using this as an index of heat resistance.

In other words, as indicated in the experiment results shown in FIG. 4, allowing 10 minutes or more are allowed to pass after decompression treatment (imparting hydrostatic pressure variation by decompression and allowing a predetermined resting time to elapse) causes the hydrophobicity of the boundary surface of the spore coat to be reduced, causes water permeability to increase, and facilitates penetration of water and heat to the spore interior, thereby achieving sterilization and inactivation of spores.

As indicated earlier, turbidity is rapidly reduced after decompression, as shown in FIG. 4. The present invention is capable of performing sufficient sterilization or inactivation by sufficiently reducing the heat resistance of heat-resistant spores in a fixed period of time (resting time) following decompression, and thereafter performing heat sterilization. In other words, in view of the effect of decompression treatment after high-pressure treatment and hydrostatic pressure variation resulting from the decompression treatment in order to enhance sterilization effect with pressure and temperature in an easily usable range without dependence on high pressure and high temperature, which have been conventionally held to allow sterilization by the independent effects of decompression treatment and heat sterilization treatment, the present invention solves previous problems upon it having been found that, *inter alia*, heat resistance of heat-resistant spore-forming bacteria is sufficiently reduced together with time, and that sufficient effect can be obtained with heat sterilization at a temperature level such as described above while heat resistance is low for a predetermined length of time, and the present invention provides an innovative method for sterilization or inactivation treatment of heat-resistant spore-forming bacteria that demonstrates a sufficient sterilization effect in a reliable manner and that can be implemented using simple equipment.

FIG. 5 is a graph showing the recovery time of the heat resistance of *B. cereus* spores reduced by the decompression treatment.

The experiment shown in FIG. 5 was performed using the sample strain Bacillus cereus NBRC 13494 (refined spores), suspending the spores in a 1/15-mol phosphate buffer solution (pH 7.0), and using an initial bacteria count of 3.6 × 10⁷ cfu/mL. Decompression treatment was carried out for 10 minutes at 200 MPa and 25°C, then the sample was left standing for 0, 6, 12, 18, 24, and 48 hours at 25°C, after which a heat sterilization treatment was carried out for 5 minutes at 90°C, and the surviving number of spores was calculated to evaluate heat resistance.

The experiment (heat treatment after standing at 25°C) represents the survival number of spore-forming bacteria when the sample was left standing at 25°C for each time period and then subjected to heat sterilization treatment for 5 minutes at 90°C. A considerable change in heat resistance was not observed even after the sample was left standing for 48 hours in a buffer solution, but a reduction on the order of about 3.5 was observed in comparison with the initial bacteria count when the sample was left standing for 18 hours after decompression treatment, the surviving spore count had increased 18 hours later on the order of about 2, and a recovery in heat resistance was confirmed.

Therefore, decompression treatment was carried out, after which heat sterilization treatment was carried out after waiting about 10 minutes, which is the length of time in which the turbidity immediately after decompression reaches 60% or less, thus demonstrating that a sufficient sterilization effect is reliably obtained by carrying out the heat sterilization treatment within at least 18 hours.

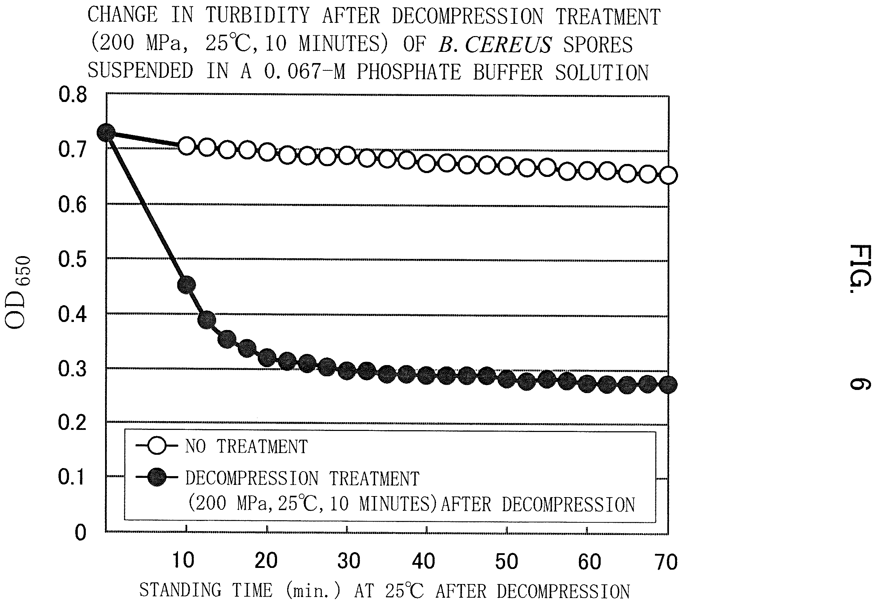

Furthermore, FIG. 6 is a graph showing change in turbidity after decompression treatment (200 MPa, 25°C, 10 minutes) of *B. cereus* spores suspended in a 0.067-M phosphate buffer solution, and reliable sterilization effect could also be confirmed from this experiment as well.

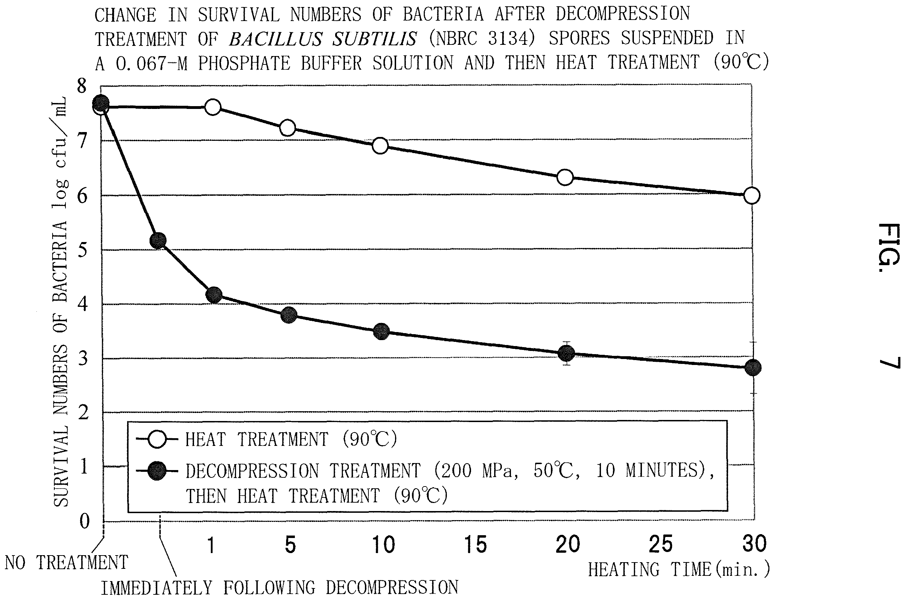

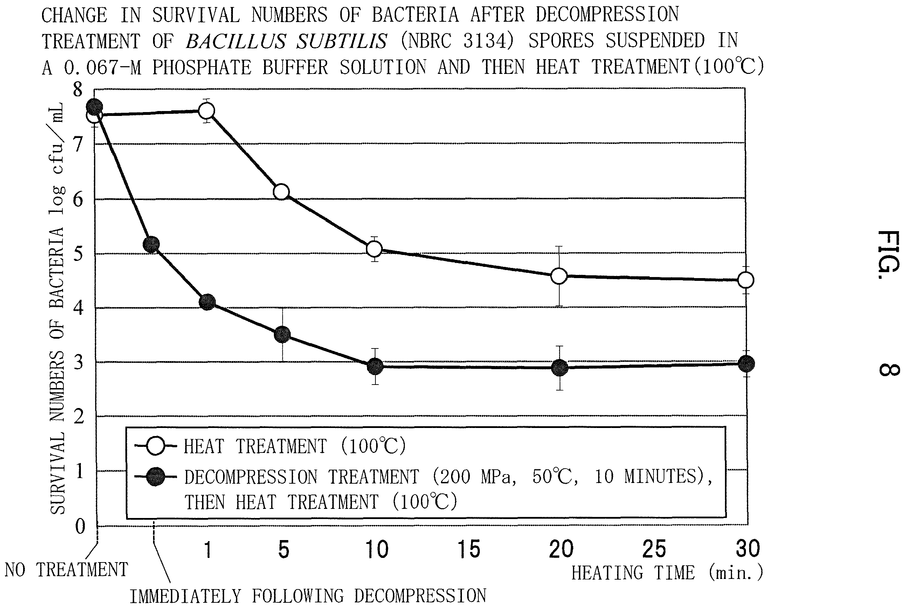

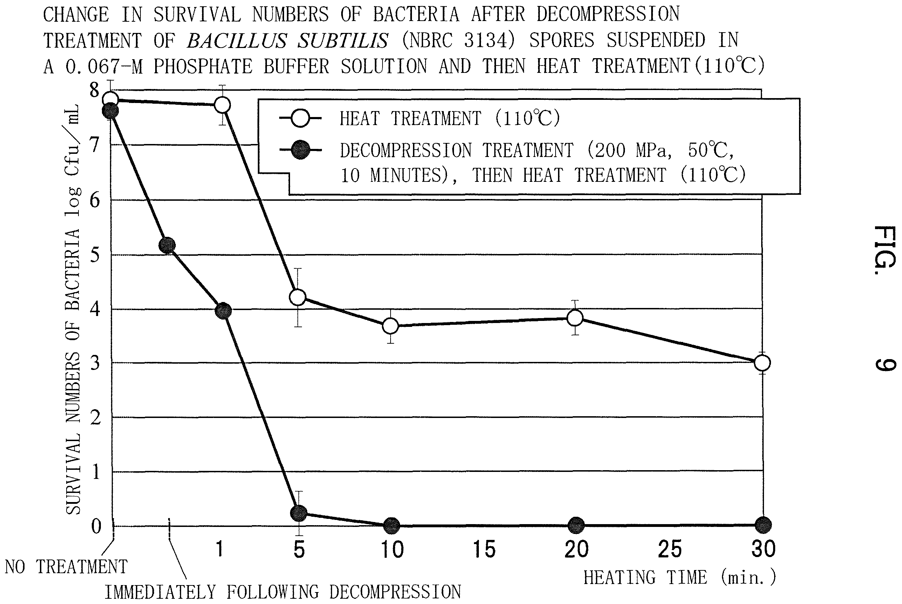

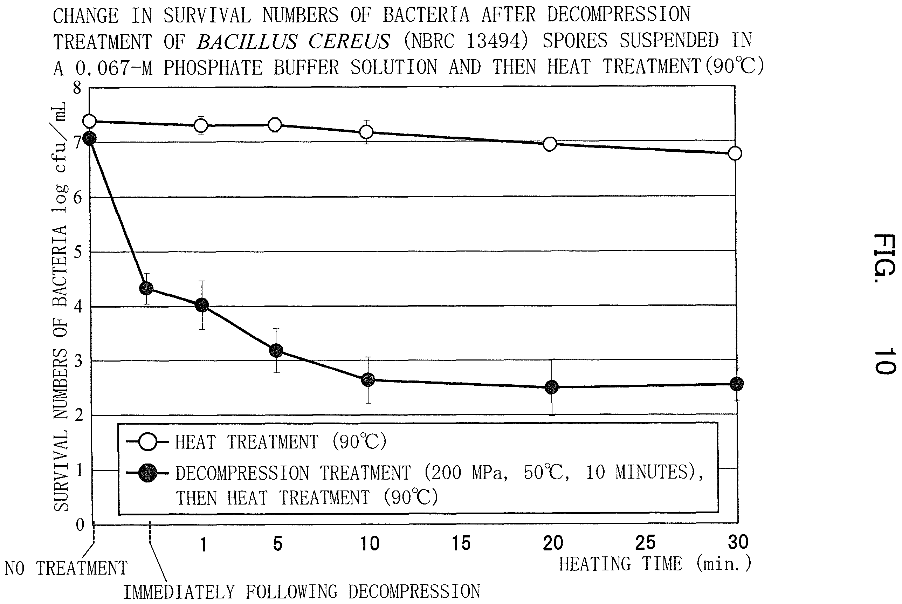

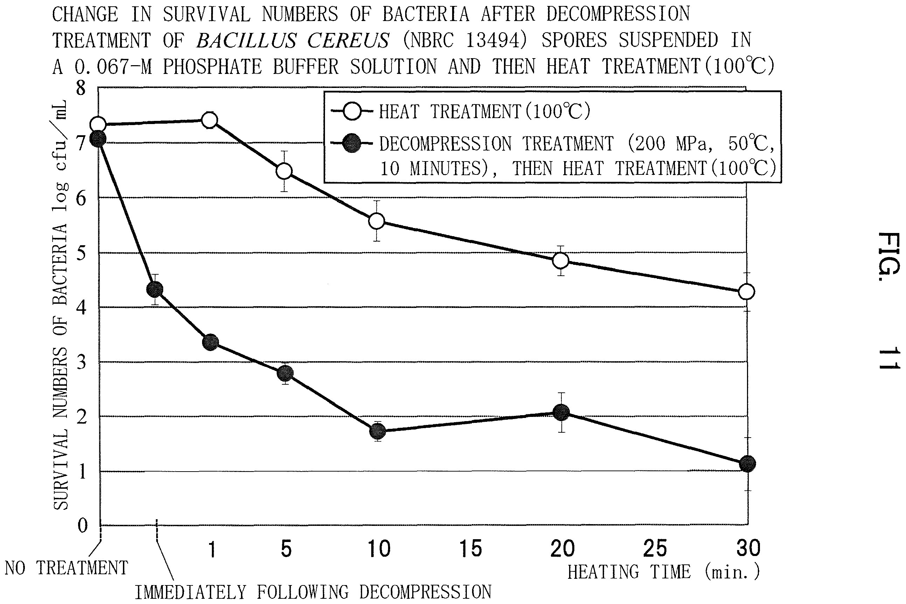

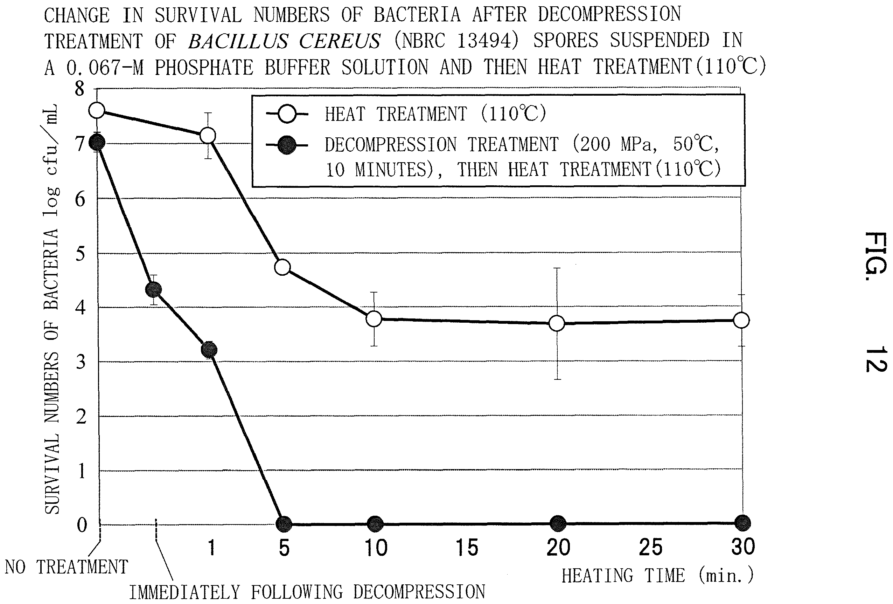

FIG. 7 is a graph showing change in bacteria survival numbers in decompression treatment of *Bacillus subtilis* (NBRC 3134) spores suspended in a 0.067-M phosphate buffer solution, and in a subsequent heat treatment (90°C). FIG. 8 is a graph showing change in bacteria survival numbers in decompression treatment of *Bacillus subtilis* (NBRC 3134) spores suspended in a 0.067-M phosphate buffer solution, and in a subsequent heat treatment (100°C). FIG. 9 is a graph showing change in bacteria survival numbers in decompression treatment of *Bacillus subtilis* (NBRC 3134) spores suspended in a 0.067-M phosphate buffer solution, and in a subsequent heat treatment (110°C). FIG. 10 is a graph showing change in bacteria survival numbers in decompression treatment of *Bacillus cereus* (NBRC 13494) spores suspended in a 0.067-M phosphate buffer solution, and in a subsequent heat treatment (90°C). FIG. 11 is a graph showing change in bacteria survival numbers in decompression treatment of *Bacillus cereus* (NBRC 13494) spores suspended in a 0.067-M phosphate buffer solution, and in a subsequent heat treatment (100°C). FIG. 12 is a graph showing change in bacteria survival numbers in decompression treatment of *Bacillus cereus* (NBRC 13494) spores suspended in a 0.067-M phosphate buffer solution, and in a subsequent heat treatment (110°C).

Reliable sterilization effect could also be confirmed from these experiments as well.

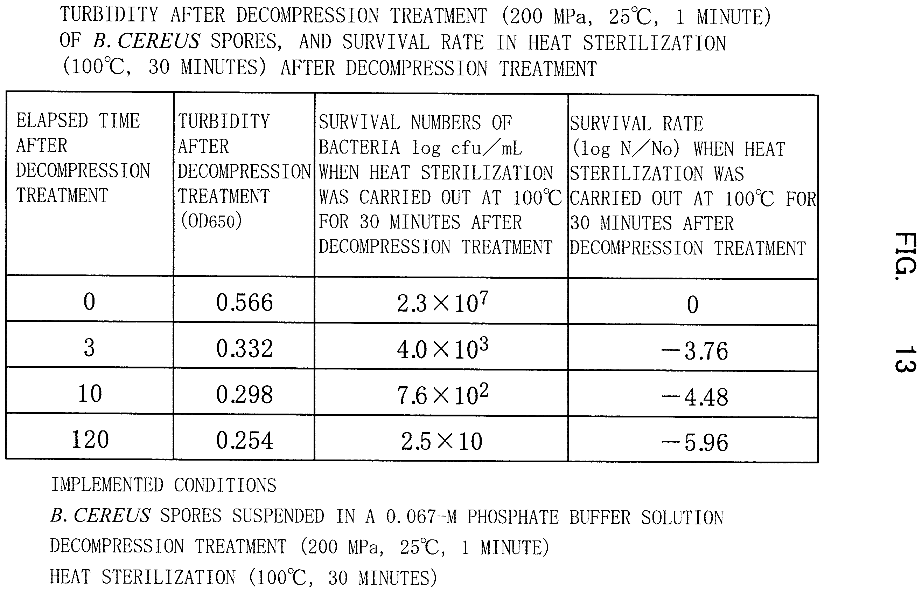

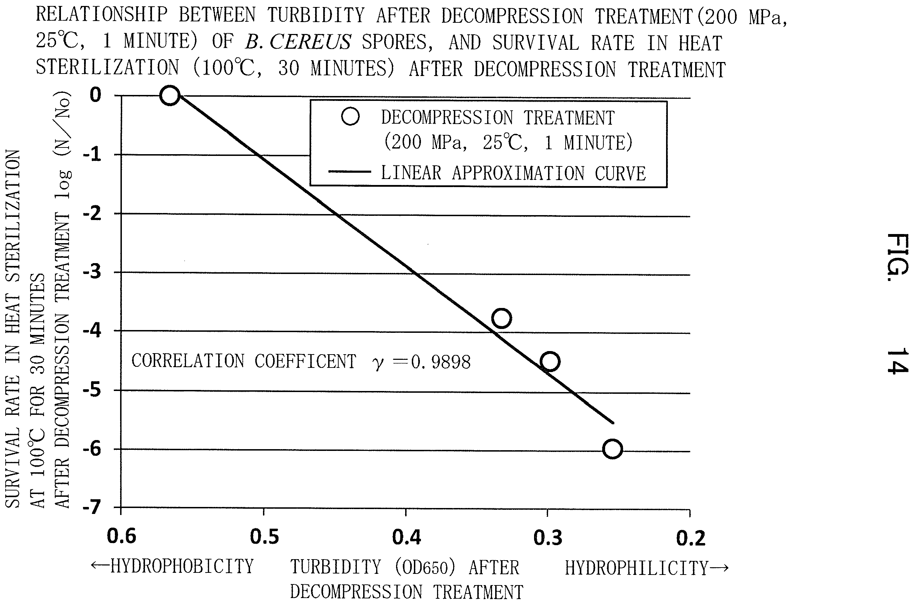

FIG. 13 is a table showing turbidity after decompression treatment (200 MPa, 25°C, 1 minute) of *B. cereus* spores, and the survival rate in heat sterilization (100°C, 30 minutes) after decompression treatment. FIG. 14 is a graph showing the relationship between turbidity after decompression treatment (200 MPa, 25°C, 1 minute) of *B. cereus* spores, and the survival rate in heat sterilization (100°C, 30 minutes) after decompression treatment.

It was confirmed from these experiment results that a reduction in turbidity after decompression treatment correlates with the survival rate of bacteria in heat sterilization treatment after decompression treatment.

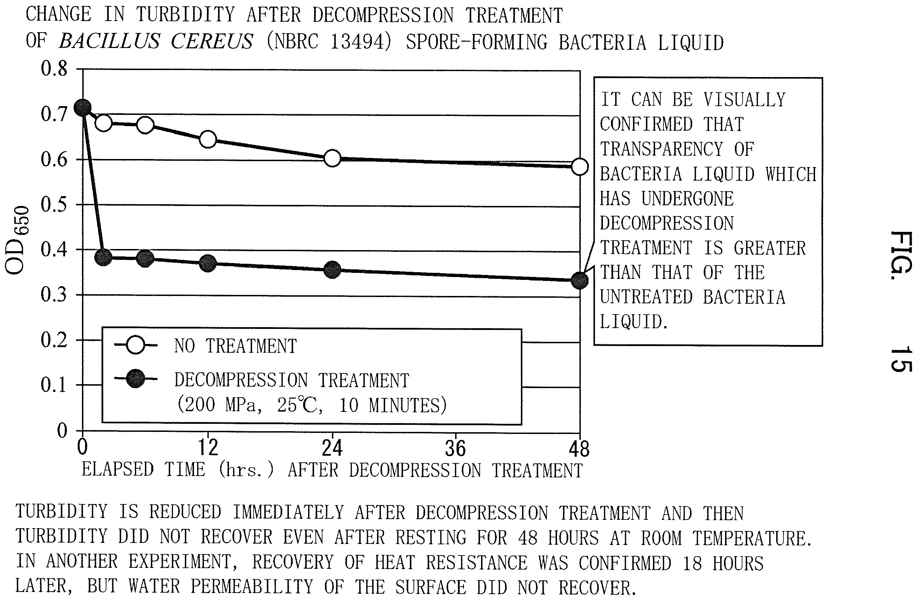

FIG. 15 is a graph showing the change in turbidity after decompression treatment of *Bacillus cereus* (NBRC 13494) spore-forming bacteria liquid.

From the results of this experiment, it was found that turbidity is reduced immediately after decompression treatment and then turbidity does not recover even after resting for 48 hours at room temperature. In a separate experiment, heat resistance was found to recover after 18 hours, though surface water permeability did not recover.

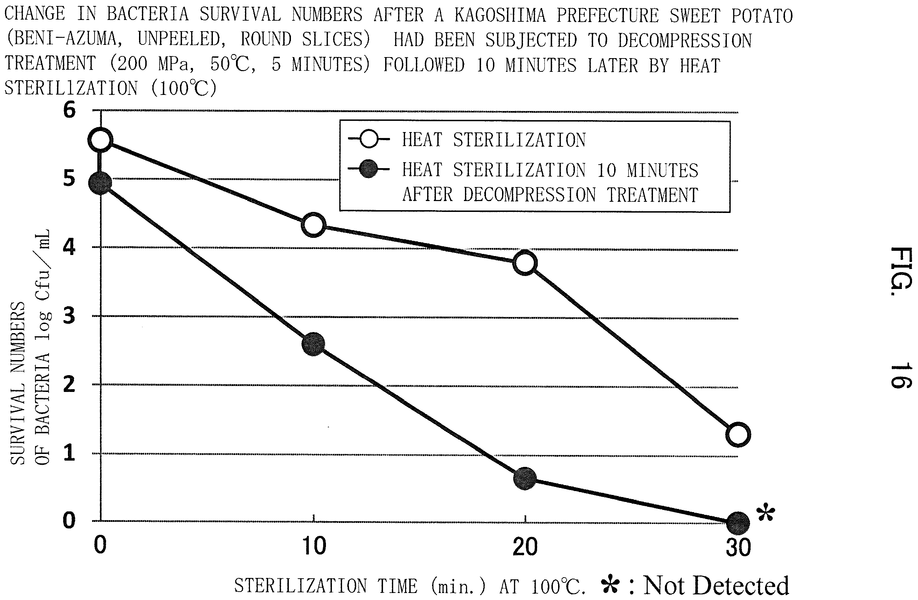

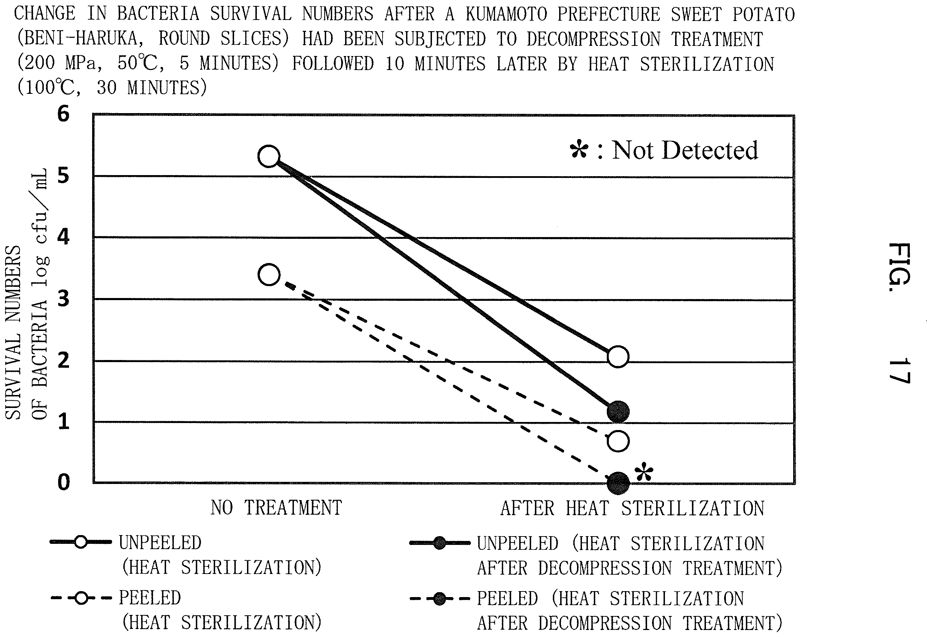

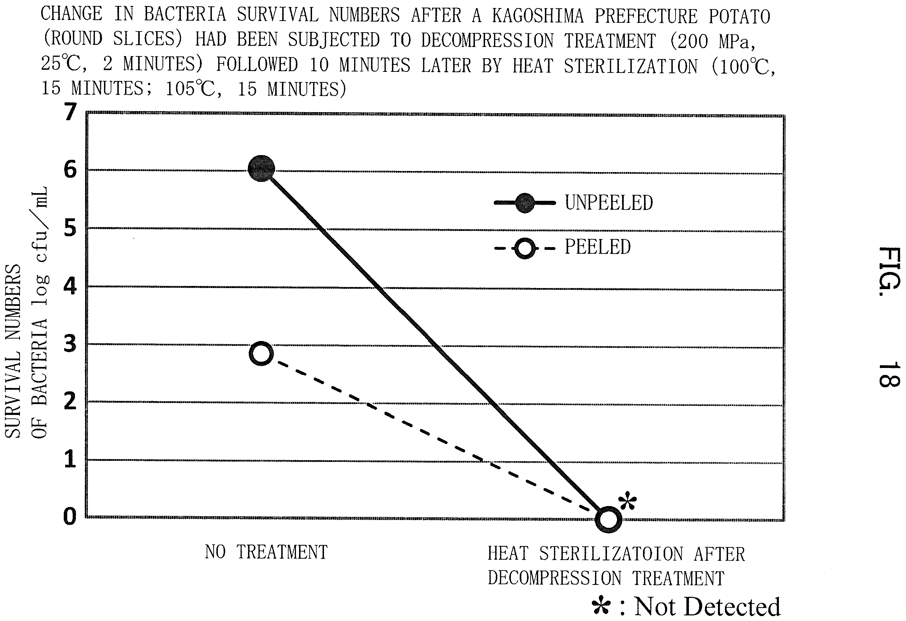

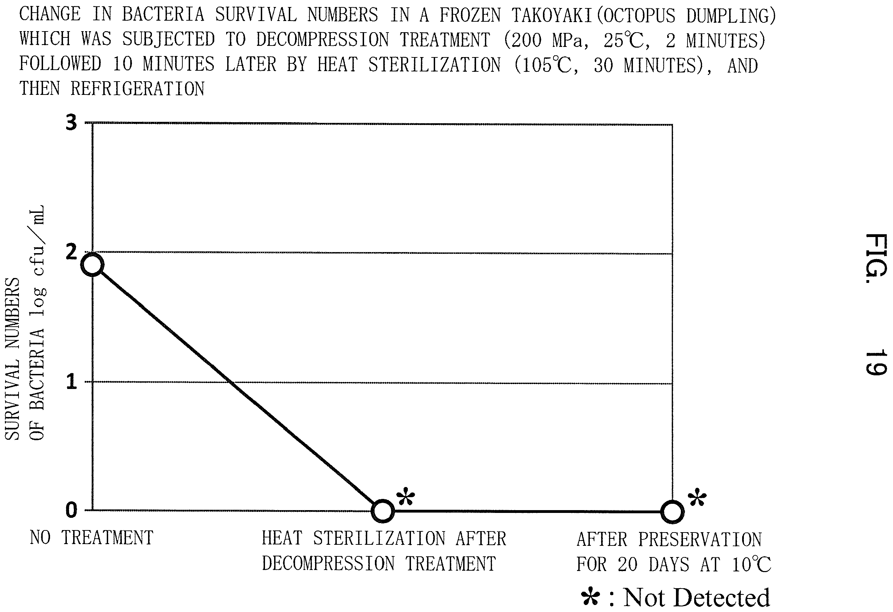

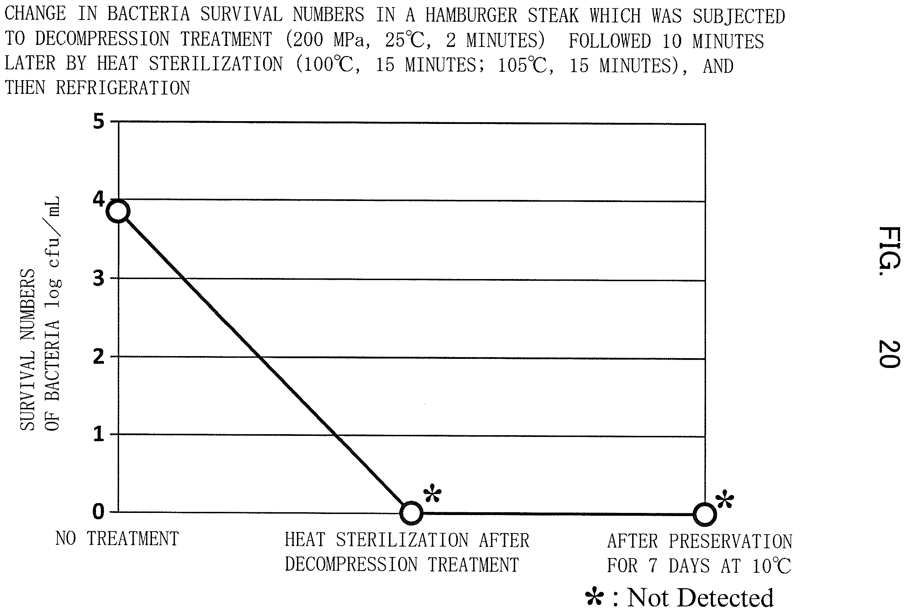

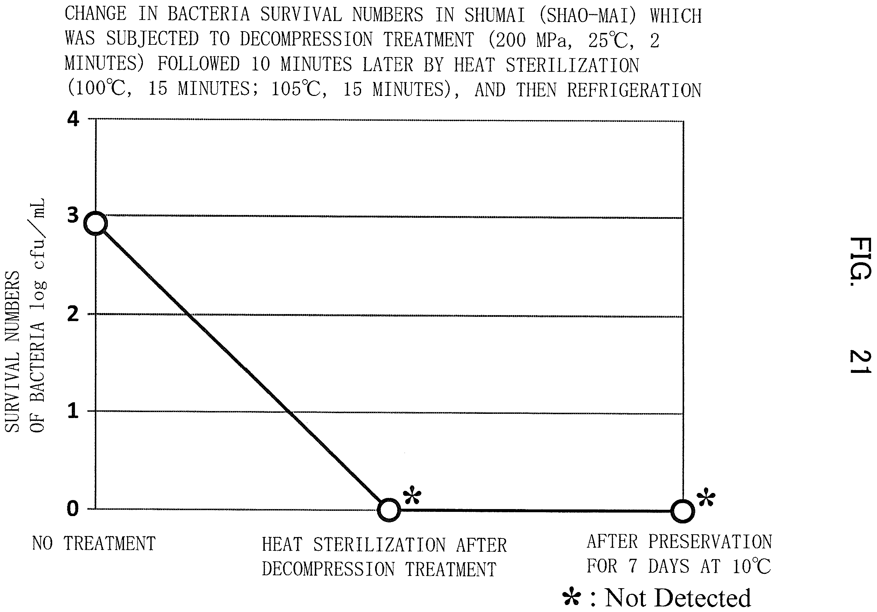

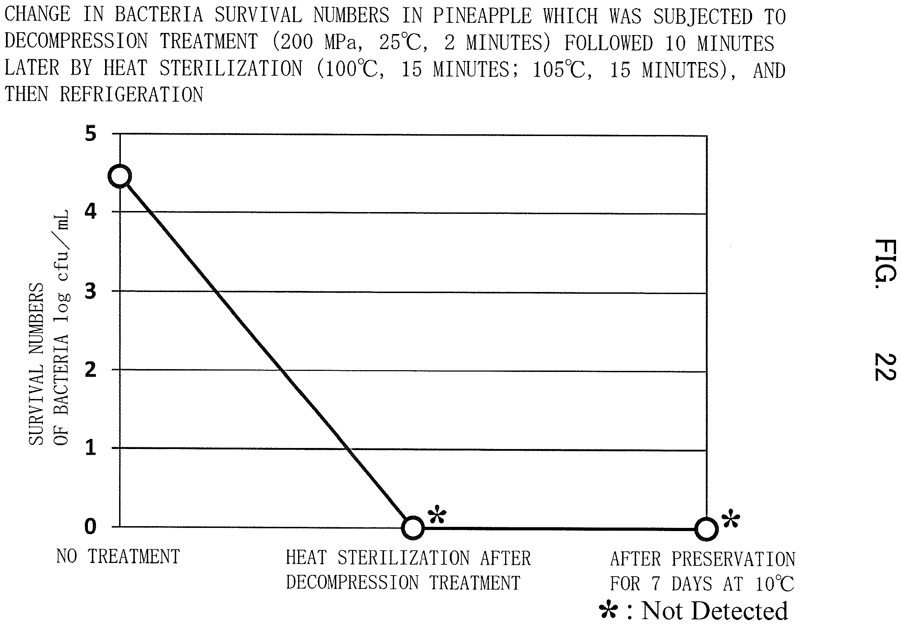

Further examples were carried out using agricultural and marine products, as well as processed foods as objects, and the resting time after decompression treatment was set to 10 minutes, i.e., examples in which heat sterilization treatment was carried out after 10 minutes had elapsed (after waiting 10 minutes). FIG. 16 is a graph showing change in bacteria survival numbers after a Kagoshima Prefecture sweet potato (beni-azuma, unpeeled, round slices) had been subjected to decompression treatment (200 MPa, 50°C, 5 minutes) followed 10 minutes later by heat sterilization (100°C). FIG. 17 is a graph showing change in bacteria survival numbers after a Kumamoto Prefecture sweet potato (beni-haruka, round slices) had been subjected to decompression treatment (200 MPa, 50°C, 5 minutes) followed 10 minutes later by heat sterilization (100°C, 30 minutes). FIG. 18 is a graph showing change in bacteria survival numbers after a Kagoshima Prefecture potato (round slices) had been subjected to decompression treatment (200 MPa, 25°C, 2 minutes) followed 10 minutes later by heat sterilization (100°C, 15 minutes; 105°C, 15 minutes). FIG. 19 is a graph showing change in bacteria survival numbers in a frozen takoyaki (octopus dumpling) which was subjected to decompression treatment (200 MPa, 25°C, 2 minutes) followed 10 minutes later by heat sterilization (105°C, 30 minutes), and then to refrigeration. FIG. 20 is a graph showing change in bacteria survival numbers in a hamburger steak which was subjected to decompression treatment (200 MPa, 25°C, 2 minutes) followed 10 minutes later by heat sterilization (100°C, 15 minutes; 105°C, 15 minutes), and then to refrigeration. FIG. 21 is a graph showing change in bacteria survival numbers in shumai (shao-mai) which was subjected to decompression treatment (200 MPa, 25°C, 2 minutes) followed 10 minutes later by heat sterilization (100°C, 15 minutes; 105°C, 15 minutes), and then to refrigeration. FIG. 22 is a graph showing change in bacteria survival numbers in pineapple which was subjected to decompression treatment (200 MPa, 25°C, 2 minutes) followed 10 minutes later by heat sterilization (100°C, 15 minutes; 105°C, 15 minutes), and then to refrigeration.

Sufficient sterilization effect was also confirmed to be demonstrated from these experiments as well.

The present invention is not limited to the present examples, and the specific configuration of the constituent features can be designed, as appropriate.

## Claims

1. A method for sterilizing or inactivating heat-resistant spore-forming bacteria, **characterized in that**, as a pretreatment of a heat sterilization treatment, a high-pressure treatment is carried out in which a hydrostatic pressure of 50 MPa or more and 200 MPa or less is applied to an object under a temperature of 50°C or less, a decompression treatment for decompression is carried out, after which the object having undergone the decompression treatment is subjected to the heat sterilization treatment, in which a temperature of at least 121°C or less, which is a temperature of 50°C to 80°C above the initial temperature, is maintained for 10 minutes or longer, after 10 minutes or more have elapsed and before 18 hours or more have elapsed after a variation in hydrostatic pressure has occurred by the decompression.

2. The method for sterilizing or inactivating heat-resistant spore-forming bacteria according to claim 1, **characterized in that** the time at which the turbidity of a spore suspension, which is an index of heat resistance after the decompression treatment, is 60% or less of the value immediately after the decompression is ascertained as a resting time by measurement in advance, a pause is made until the resting time has elapsed immediately after the decompression, and the heat sterilization treatment is carried out.

3. The method for sterilizing or inactivating heat-resistant spore-forming bacteria according to claim 1 or 2, **characterized in that** the object is harvested agricultural and marine products, processed foods processed using these products, or animal organs, blood, animal cell tissue, plant cell tissue, or seeds.
